(19) Europäisches Patentamt
European Patent Office
Office européen des brevets

(11) EP 1 092 418 B1

(12) EUROPÄISCHE PATENTSCHRIFT

(45) Veröffentlichungstag und Bekanntmachung des Hinweises auf die Patenterteilung:
**19.01.2005 Patentblatt 2005/03**

(51) Int Cl.[7]: **A61K 7/06**

(21) Anmeldenummer: **00121718.1**

(22) Anmeldetag: **05.10.2000**

(54) **Festigende Haarreinigungsmittel**

Hair styling shampoos

Shampooings contenant un fixateur

(84) Benannte Vertragsstaaten:
**AT BE CH CY DE DK ES FI FR GB GR IE IT LI LU MC NL PT SE**

(30) Priorität: **14.10.1999 DE 19949516**

(43) Veröffentlichungstag der Anmeldung:
**18.04.2001 Patentblatt 2001/16**

(73) Patentinhaber: **Henkel Kommanditgesellschaft auf Aktien**
**40589 Düsseldorf-Holthausen (DE)**

(72) Erfinder:
- **Gassenmeier, Thomas Otto, Dr.**
  **40229 Düsseldorf (DE)**
- **Schröder, Josefine**
  **41812 Erkelenz (DE)**
- **Busch, Peter, Dr.**
  **40699 Erkrath (DE)**

(56) Entgegenhaltungen:
**EP-A- 0 283 817**
**EP-A- 0 463 780**
**EP-A- 0 572 768**
**EP-A- 0 574 696**
**WO-A-91/15185**
**WO-A-97/07782**
**US-A- 5 087 443**
**US-A- 5 553 630**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Haarshampoos mit einem Gehalt an wasserlöslichen, schäumenden Tensiden, die ein darin dispergiertes filmbildendes Polymerisat enthalten und dadurch dem damit gewaschenen Haar eine erhöhte Festigkeit und der Frisur einen verbesserten Halt verleihen.

Haarfestigungsshampoos sind in der Patentliteratur häufig beschrieben worden. Diese Shampoos enthalten übliche filmbildende Polymere, die durch ein geeignetes Lösungsmittel in der Shampoozusammensetzung solubilisiert werden. Solche Shampoos werden z. B. in WO 97/07782 A1 oder in WO 91/15185 A1 beschrieben. Ein anderer Typ von festigenden Shampoos enthält Polymere, die sich in Wasser oder in dem Tensidsystem des Shampoos lösen lassen. Solche Festigungsshampoos sind z. B. aus US 5,391,386 A1 und aus EP 0283817 B1 bekannt.

Es wurde nun aber festgestellt, daß auch wasserunlösliche Polymerisate, die als feinteilige Dispersion in Wasser beziehungsweise in dem Shampoo vorliegen, eine überraschend gute festigende Wirkung auf die damit gewaschenen Haare ausüben. Diese Wirkung scheint durch die Gegenwart von Tensiden in einer synergistischen Weise gesteigert zu werden.

Gegenstand der Erfindung sind daher festigende Haarreinigungsmittel in Form von wäßrigen Zubereitungen mit einem Gehalt von

| | |
|---|---|
| 1-30 Gew.% | eines wasserlöslichen schäumenden Tensids und |
| 0,1-10 Gew. % | eines darin dispergierten filmbidenden Polymers, welches ein Homo- oder Copolymerisat aus Monomereinheiten der Formel I darstellt, |

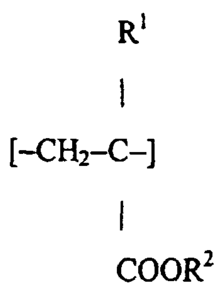

(I)

in der $R^1$ Wasserstoff oder eine Methylgruppe und $R^2$ Wasserstoff oder eine Alkylgruppe mit 1-8 C-Atomen ist,

dadurch gekennzeichnet, daß nicht mehr als 60 Mol % der Monomereinheiten als $R^2$ Wasserstoff enthalten.

Als filmbildende Polymere eignen sich alle von Acrylsäure und Methacrylsäure und deren Estern mit $C_1$-$C_8$-Alkoholen abgeleiteten Polymerisate, die bis zu 60 Mol % an monomeren Acryl- und Methacrylsäureeinheiten mit freier Carboxylgruppe enthalten können. Bevorzugt sind solche Polymerisate, deren mittleres Molekulargewicht höher ist als $(63-x)10^4$, wobei x der Anteil der Monomeren in Mol % ist, in denen $R^2$ Wasserstoff ist.

Besonders bevorzugt eignen sich dabei solche Haarreinigungsmittel, die als filmbildende Polymere ein Copolymerisat aus Monomereinheiten der Formel I enthalten, in der $R^1$ Wasserstoff oder eine Methylgruppe und $R^2$ eine Methyl- oder Ethylgruppe ist und deren mittleres Molekulargewicht M oberhalb von 700000 D (Dalton) liegt. Solche Polymeren sind in Form einer wäßrigen Dispersion unter der Bezeichnung Eudragit® NE 30 D (Röhm) als Acrylharz-Lack zum Überziehen von Arzneistoff-Pellets oder Granulaten im Handel.

Eine weitere bevorzugte Ausführung der Erfindung betrifft festigende Haarreinigungsmittel, die als filmbildende Polymere ein Copolymersisat aus Monomereinheiten der Formel I enthalten, in der $R^1$ Wasserstoff oder eine Methylgruppe und $R^2$ Wasserstoff, eine Methyl- oder eine Ethylgruppe ist, wobei 40-60 Mol % der Monomeren als $R^2$ Wasserstoff enthalten und deren mittleres Molgewicht oberhalb von 100 000, bevorzugt bei 130 000 bis 300 000 liegt. Solche Copolymerisate sind z. B. unter der Bezeichnung Eudragit® L100 oder L-100-55 oder als Dispersion unter der Bezeichnung Eudragit® L30D-55 im Handel erhältlich.

Die dispergierten filmbildenden Polymeren, welche ein Homo- oder Copolymerisat aus Monomereneineiten der Formel I darstellen, eignen sich hervorragend zur Verwendung als haarfestigende Komponente in wäßrigen Haarreinigungsmitteln mit einem Gehalt an 1-30 Gew.% wasserlöslicher, schäumender Tenside. Sie haben keinen negativen Einfluß auf das Schaumvermögen und die Reinigungswirkung der Tenside und hinterlassen das Haar nach der Wäsche in einem leicht kämmbaren und formbaren und nach dem Trocknen in einem gefestigten, elastischen Zustand.

Als wasserlösliche, schäumende Tenside sind insbesondere anionische Tenside geeignet wie sie üblicherweise zur Formulierung von Shampoos eingesetzt werden. Solche anionischen Tenside sind z. B.

- Alkylsulfate und Alkylpolyglycolethersulfate der Formel $R^3O-(CH_2CH_2O)_x-SO_3H$, in der $R^3$ eine bevorzugt lineare Alkylgruppe mit 10 bis 16 C-Atomen und x=0 oder eine Zahl von 1-10 ist,
- Sulfobernsteinsäuremonoester von Alkylpolyglycolethern mit 10-16 C-Atomen in der Alkylgruppe und 1-10 Glycolethergruppen,
- Ethercarbonsäuren der Formel $R^4O(CH_2CH_2O)_x-CH_2-COOH$, in der $R^4$ eine lineare Alkylgruppe mit 10-16 C-Atomen und x=0 oder eine Zahl von 1-10 ist,
- lineare Alkansulfonate mit R 12-18 C-Atomen,
- lineare Alpha-Olefinsulfonate mit 12-18 C-Atomen,
- Acylisethionate mit 10-18 C-Atomen in der Acylgruppe,
- Acylsarkoside mit 10 bis 18 C-Atomen in der Acylgruppe,
- Acyltauride mit 10 bis 18 C-Atomen in der Acylgruppe,
- Citronensäure- oder Weinsäuremonoester von Alkylpolyglycolethern mit 10-16 C-Atomen in der Alkylgruppe und 1-10 Glycolethergruppen und
- Acylierte Proteinhydrolysate mit 12-18 C-Atomen in der Acylgruppe,

jeweils ins Form der gut wasserlöslichen Alkali-, Magnesium-, Ammonium oder Alkanolammoniumsalze.

Weitere, gut schäumende Tenside sind auch bestimmte amphotere, zwitterionische und nichtionogene Tenside. Eine bevorzugte Stellung unter den nichtionogenen Tensiden haben vor allem die Alkyl-(oligo)glucoside, die durch Umsetzung von Alkoholen mit 8 bis 16 C-Atomen mit z. B. Butylglucosid durch Transacetalisierung oder durch direkte Acetalisierung aus Glucose und Fettalkohol zugänglich sind und der Formel $RO-(Z)_x$ entsprechen, in der R eine $C_8$-$C_{16}$-Alkyl- oder Alkenylgruppe und Z einen Monosaccharidrest, insbesondere Glucose, und x dessen mittleren Oligomerisationsgrad, eine Zahl von 1-5, bevorzugt von 1 bis 2 darstellt.

Bevorzugt sind als wasserlösliche, schäumende Tenside anionische Tenside, Alkyl(oligo)glucosid-Tenside oder ein Gemisch solcher Tenside enthalten. Weiterhin sind solche Haarreinigungsmittel bevorzugt, die als anionisches Tensid ein Alkylsulfat- oder Alkylpolyglycolethersulfat-Tensid mit 10-16 C-Atomen in der Alkylgruppe und bis zu 10 Glycolethergruppen in Form eines Alkali-, Magnesium-, Ammonium- oder Alkanolammoniumsalzes enthalten.

Die zwitterionischen und amphoteren Tenside werden bevorzugt in Kombination mit starkschäumenden anionischen Tensiden oder Alkyl-(oligo)glucosiden eingesetzt und dienen dazu, die Hautverträglichkeit zu verbessern und die Viskosität und das Schäumvermögen der Kombination zu steigern.

Als zwitterionische Tenside werden solche oberflächenaktiven Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer $C_{8-18}$ -Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat und das Kokosacylaminoethy laminopropionat.

Weitere, bevorzugt in Kombination mit anionischen Tensiden verwendete nichtionogene Tenside sind z. B.

- Anlagerungsprodukte von 2-30 Mol Ethylenoxid und/oder von 1-5 Mol Propylenoxid an lineare Fettalkohole mit 8-18 C-Atomen, an Fettsäuren mit 12-18 C-Atomen, an Fettsäuremonoglyceride von $C_{12}$-$C_{18}$-Fettsäuren, an Sorbitanmonofettsäureester von Fettsäuren mit 12-18 C-Atomen, an Fettsäurealkanolamide, an Methylglucosid-Fettsäureester, an gehärtetes Rizinusöl und an andere Lipide mit alkoxylierbaren funktionellen Gruppen.

- Aminoxid-Tenside, z. B. Alkylaminoxide mit 12-18 C-Atomen und Acylamidopropyl-dimethylaminoxid mit 12-18 c-Atomen in der Acylgruppe.

Auch selbst nicht wasserlösliche Tenside, die aber in Gegenwart wasserlöslicher Tenside solubilisiert werden und dann sowohl zur Viskosität und zur verdickbarkeit durch Elektrolyte als auch zur Feinblasigkeit und Cremigkeit des Schaumes beitragen, könne in Mengen bis zu 5 Gew.% in den erfindungsgemäßen Haarreinigungsmitteln enthalten sein. Solche Produkte sind z. B.

- Fettsäuremonoethanolamide, Fettsäurediethanolamide und Fettsäuremonoisopropanolamide von $C_{12}$-$C_{18-}$ Fettsäuren,
- Fettsäurepartialgyceride (Monoglyceride und Mono-/Diglyceridgemische) und
- Sorbitanmono- und difettsäureester

Die erfindungsgemäßen Haarreinigungsmittel enthalten, die filmbildenden Polymeren in feindispergierter Form. Die Einarbeitung ist in der Regel kein Problem, da die Polymeren bereits als Dispersion oder Pulver im Handel sind und sich ohne Schwierigkeiten in der Tensidphase verteilen lassen. Da organische Lösungsmittel nicht nur nicht erforderlich, sondern in Haarshampoos auch aus Gründen der dadurch verminderten Schäumkraft unerwünscht sind, sind die erfindungsgemäßen Haarreinigungsmittel von organischen Lösemitteln im wesentlichen frei. Mit "im wesentlichen" soll zum Ausdruck gebracht werden, daß Spuren von organischen Lösungsmitteln, die durch andere Rohstoffe, z. B. Farb- und Duftstoffe oder Konservierungsmittel eingetragen werden, nicht ausgeschlossen werden sollen. In der Regel werden solche Mengen an organischen Lösungsmitteln unterhalb von 2,5 Gew.-%, bezogen auf das gesamte Mittel, liegen.

Daneben können die erfindungsgemäßen Haarreinigungsmittel alle üblichen Hilfs- und Zusatzstoffe enthalten, die in Shampoos üblicherweise verwendet werden. Dazu gehören z. B.

- emulgierte Ölkomponenten, bevorzugt in mikroemulgierter Form mit Tröpfchengrößen unter 100 nm. Geeignete Ölkomponenten sind z. B. Pflanzenöle, synthetische Triglyceridöle, Fettsäurester, Silikone, Paraffinöle, Squalan und synthetische Kohlenwasserstoffe wie z. B. Dioctylcyclohexan,

- emulgierte Wachse, bevorzugt in Form von Nanodispersionen,

- wasserlösliche nichtionische Polymere wie z. B. Polyvinylalkohol, Polyethylenglycole, Polyacrylamide,

- Verdickungsmittel wie z. B. Guar-Gum, Alginate, Xanthan-Gum, Cellulosederivate wie z. B. Methylcellulose, Hydroxyethylcellulose, Carboxymethylcellulose, lösliche Stärkederivate,

- wasserlösliche anionische Polymere wie z. B. die löslichen Salze von Polyacrylsäure und Polymethacrylsäure und deren Copolymeren mit Acryl- und Methacrylsäureestern,

- wasserlösliche zwitterionische und amphotere Polymeriate,

- wasserlösliche kationische Polymere, z. B. quaternierte Hydroxyethylcellulose (durch Umsetzung mit Epoxypropyltrimethylammoniumchlorid), Dimethyldialkylammoniumchlorid-Polymere und deren Copolymere mit Acrylamid, Dimethylaminoethylmethacrylat-Vinylpyrrolidon-Copolymere und deren quaternierte Derivate und Vinylpyrrolidon-Imidazoliniummethochlorid-Copolymere,

- haarkonditionierende und strukturierende Zusätze wie z. B. Phospholipide (Lecithin), Silikonöle, Glucose, Maleinsäure,

- Proteinhydrolysate, wie z. B. Collagenhydrolysate oder Weizenproteinhydrolysate,

- haarkosmetische Wirkstoffe wie z. B. Vitamine, Panthenol, Allantoin, Pyrrolidoncarbonsäure, Cholesterin, Antischuppenwirkstoffe wie z. B. Salicylsäure, Zink Omadine oder Piroctone Olamine,

- Quell- und Penetrationshilfsmittel wie z. B. Guanidin, Harnstoff, Hydrogencarbonate,
- pH-Einstellmittel wie z. B. Citronensäure, Milchsäure und Puffergemische,
- Komplexbildner wie z. B. EDTA, NTA, Acetophosphonsäuren,
- Konservierungsmittel und Antioxydantien,
- Farbstoffe, Trübungsmittel und Perlglanzmittel,
- Duftstoffe und Deodorantien,

Die folgenden Beispiele sollen den Erfindungsgegenstand näher erläutern:

Beispiele

Es wurden die folgenden Prüfshampoos hergestellt

| | 1 | 2 | V1 | V2 |
|---|---|---|---|---|
| Texapon® NSO | 34,3 | - | 34,3 | - |
| Plantacare® 818 | - | 19,23 | - | 19,23 |
| Dehyton® K | 9,67 | 9,67 | 9,67 | 9,67 |
| Polymer A bis D (AS) | 1,0 | 1,0 | - | - |
| Wasser mit Citronen-Säure bis pH=4 | ad 100 | ad 100 | ad 100 | ad 100 |

Es wurden folgende Handelsprodukte eingesetzt:

| | |
|---|---|
| Polymer A | |
| Eudragit® NE30D: (Dispersion, 30 Gew.%AS) | Copolymerisat von Acrylsäure- und Methacrylsäureestern $R^1$=H, $CH_3$ und $R^2$= $CH_3$, $C_2H_5$ Mittleres Molgewicht: 800 000 D Glasübergangstemperatur: -8°C |
| Polymer B: | |
| Eudragit® L 100-55: (Pulver, 100 Gew.% AS) | Copolymerisat aus Acrylsäure, Methacrylsäure und deren Methyl- und Ethylestern $R^1$=H, $CH_3$ und $R^2$=H(50 Mol%) $CH_3$ und $C_2H_5$ (50 Mol%) Mittleres Molgewicht: 250 000 D Glasübergangstemperatur: 110°C |
| Polymer C: | |
| Eudragit® L 30D-55: | Wäßrige Dispersion von Eudragit L 100-55 (30 Gew.% AS) |
| Texapon® NSO: | Alkyl($C_{12/14}$)-polyglycolether (2EO)-sulfat, Na-Salz (28 Gew.% AS in Wasser) |
| Plantacare® 818: | Kokosalkyl (1,4)-glucosid (50 Gew.% AS in Wasser) |
| Dehyton® K: | Kokosacylamidopropyl-dimethylammonium-acetobetain (30 Gew.%AS + 5 Gew.% NaCl in Wasser) |

Verfahren zur Bestimmung der Festiger-Wirkung (curl-retention)

Test-Haarsträhnen wurden mit einer Alkylethersulfat-Lösung bei pH= 6,5 gereinigt, gespült und getrockenet.
Die so erhaltenen Strähnen wurden angefeuchtet und mit dem zu prüfenden Podukt behandelt und nach 5

Minuten mit Wasser ausgespült.

Anschließend wurden die Prüfsträhnen an einem Spanngewicht befestigt und auf Wickler gewickelt. Nach zwei Stunden wurden die Haarsträhnen vorsichtig von den Wicklern entfernt und auf Bretter mit Längenskalierung geklemmt. Die Bretter wurden senkrecht in einen Klimaschrank gestellt, in dem Temperatur und Luftfeuchtigkeit regulierbar sind. Die Längenänderung der gewellten Prüfsträhne wurde nach 2 Stunden und nach 24 Stunden bei 60 % relativer Luftfeuchte beobachtet.

Für jede Test-Strähne wurde der curl-retention-Wert (C.R.) wie folgt ermittelt:

$$C.R. = \frac{L\text{-}Lt}{L\text{-}Lo} \times 100\ [\%]$$

Lo = Länge der gelockten Strähne nach der Entfernung der Wickler (zur Beginn der Klimatisierung) in cm

Lt = Länge der gelockten Strähne nach Klimalagerung in cm

L = Länge der gestreckten Haarsträhne in cm. Dieser Wert wird zuletzt ermittelt, um die Bestimmung von Lo und Lt nicht zu stören.

Um statistisch gesicherte Werte zu erhalten, wurde jedes Prüfprodukt an fünf Haarsträhnen getestet und jeweils der Mittelwert gebildet.

Ergebnisse:

Die Werte der Vergleichprodukte V1 und V2 (ohne filmbildende Polymere) unterscheiden sich nur wenig vom Wasserwert.

Die Verbesserung des C.R. Wertes in % bezogen auf den Wasserwert (ohne filmbildendes Polymer) sind in der folgenden Tabelle angegeben:

| | **2 h, 60 % relative Luftfeuchte** | | **24 h, 60% relative Luftfeuchte** | |
|---|---|---|---|---|
| **Prüfvorprodukt** | **C.R.** | **% Verbesserung** | **C.R.** | **% Verbesserung** |
| | | | | |
| Wasser | 47,5 | — | 40,1 | — |
| V1 (pH=4) | 45,2 | - 4,9 % | 38,9 | - 3 % |
| V2 (pH=4) | 47,2 | - 0,7 % | 39,3 | - 2 % |
| | | | | |
| Wasser | 43,1 | — | 36,2 | — |
| Wasser+1 Gew.%A | 45,3 | +5,1% | 39,0 | +7,7% |
| Wasser+1 Gew.%C | 45,7 | +6,0% | 39,5 | +9,1% |
| | | | | |
| Wasser | 39,0 | — | 35,7 | — |
| 1 (Polymer A) | 56,8 | + 45,6 % | 48,5 | + 35,8 % |
| 1 (Polymer B) | 50,0 | + 28,2 % | 44,6 | + 24,9 % |
| 1 (Polymer C) | 56,1 | + 43,8 % | 48,8 | + 36,7 % |
| | | | | |
| Wasser | 44,1 | — | 35,7 | — |
| 2 (Polymer A) | 56,8 | + 28,8 % | 53,4 | + 50,4 % |
| 2 (Polymer C) | 55,3 | + 25,4% | 49,9 | + 39,8% |

## Patentansprüche

1. Festigendes Haarreinigungsmittel in Form einer wäßrigen Zubereitung mit einem Gehalt von

| | |
|---|---|
| 1-30 Gew.% | eines wasserlöslichen, schäumenden Tensids und |
| 0,1-10 Gew.% | eines darin dispergierten filmbildenden Polymers, welches ein Homo- oder Copolymerisat aus Monomereinheiten der Formel (I) darstellt, |

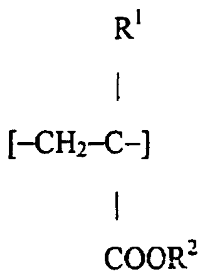

(I)

in der $R^1$ Wasserstoff oder eine Methylgruppe und $R^2$ Wasserstoff oder eine Alkylgruppe mit 1-8 C-Atomen ist,

**dadurch gekennzeichnet, daß** nicht mehr als 60 Mol % der Monomeren als $R^2$ Wasserstoff enthalten.

2. Festigendes Haarreinigungsmittel nach Anspruch 1, **dadurch gekennzeichnet, daß** als filmbildendes Polymer ein Copolymerisat aus Monomereinheiten der Formel I enthalten ist, in der $R^1$ Wasserstoff oder eine Methylgruppe und $R^2$ eine Alkyl- oder Ethylgruppe ist und das mittlere Molgewicht oberhalb von 700 000 liegt.

3. Festigendes Haarreinigungsmitel nach Anspruch 1, **dadurch gekennzeichnet, daß** als filmbildendes Polymer ein Copolymerisat aus Monomereinheiten der Formel I enthalten ist, in der $R^1$ Wasserstoff oder eine Methylgruppe und $R^2$ Wasserstoff oder eine Methyl- oder Ethylgruppe ist, **dadurch gekennzeichnet, daß** 40-60 Mol.% der Monomeren als $R^2$ Wasserstoff enthalten und das mittlere Molgewicht oberhalb von 100 000 liegt.

4. Festigendes Haareinigungsmittel nach einem der Ansprüche 1 - 3, **dadurch gekennzeichnet, dass** das Mittel organische Lösungsmittel unterhalb von 2,5 Gew.-%, bezogen auf das gesamte Mittel, enthält.

5. Festigendes Haarreinigungsmittel nach einem der Ansprüche 1-4, **dadurch gekennzeichnet, daß** als wasserlösliches, schäumendes Tensid ein anionisches Tensid, ein Alkyl-(oligo)glucosid-Tensid oder ein Gemisch solcher Tenside enthalten ist.

6. Festigendes Haarreinigungsmittel und Anspruch 5, **dadurch gekennzeichnet, daß** als anionisches Tensid ein Alkylsulfat- oder Alkylpolyglycolethersulfat-Tensid mit 10-16 C-Atomen in der Alkylgruppe und bis zu 10 Glycolethergruppen in Form eines Alkali-, Magnesium-, Ammonium oder Alkanolammoniumsalzes enthalten ist.

7. Verwendung eines dispergierten filmbildenden Polymers, welches ein Homo- oder Copolymerisat aus Monomereinheiten der Formel (I) darstellt

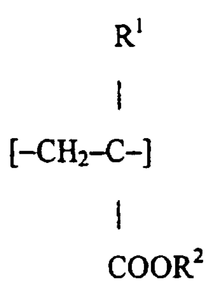

(I)

in der $R^1$ Wasserstoff oder eine Methylgruppe und $R^2$ Wasserstoff oder eine Alkylgruppe mit 1-8 C-Atomen ist und worin nicht mehr als 60 Mol.% der Monomeren als $R^2$ Wasserstoff enthalten,
als haarfestigende Komponente in einem wäßrigen Haarreinigungsmittel mit einem Gehalt von 1-30 Gew.% wasserlöslicher, schäumender Tenside.

## Claims

**1.** A strengthening hair shampoo in the form of a water-based preparation containing

1 to 30% by weight of a water-soluble foaming surfactant and, dispersed therein,
0.1 to 10% by weight of a film-forming polymer dispersed therein in the form of a homo- or a copolymer of monomer units corresponding to formula (I):

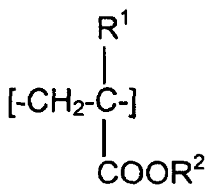

(I)

in which $R^1$ is hydrogen or a methyl group and $R^2$ is hydrogen or an alkyl group containing 1 to 8 carbon atoms, **characterized in that** no more than 60 mol-% of the monomers contain hydrogen as $R^2$.

**2.** A strengthening hair shampoo as claimed in claim 1, **characterized in that** the film-forming polymer is a copolymer of monomer units corresponding to formula I, in which $R^1$ is hydrogen or a methyl group and $R^2$ is an alkyl or ethyl group, and the average molecular weight is above 700,000.

**3.** A strengthening hair shampoo as claimed in claim 1, **characterized in that** the film-forming polymer is a copolymer of monomer units corresponding to formula I, in which $R^1$ is hydrogen or a methyl group and $R^2$ is hydrogen or a methyl or ethyl group, **characterized in that** 40 to 60 mol-% of the monomers contain hydrogen as $R^2$ and the average molecular weight is above 100,000.

**4.** A strengthening hair shampoo as claimed in any of claims 1 to 3, the shampoo as a whole, are present.

**5.** A strengthening hair shampoo as claimed in any of claims 1 to 4, **characterized in that** the water-soluble foaming surfactant is an anionic surfactant, an alkyl (oligo)glucoside surfactant or a mixture of such surfactants.

**6.** A strengthening hair shampoo as claimed in claim 5, **characterized in that** the anionic surfactant is an alkyl sulfate or alkyl polyglycol ether sulfate surfactant containing 10 to 16 carbon atoms in the alkyl group and up to 10 glycol ether groups in the form of an alkali metal, magnesium, ammonium or alkanolammonium salt.

**7.** The use of a dispersed film-forming polymer in the form of a homo- or a copolymer of monomer units corresponding to formula (I):

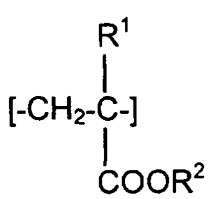

(I)

where $R^1$ is hydrogen or a methyl group and $R^2$ is hydrogen or an alkyl group containing 1 to 8 carbon atoms, no more than 60 mol-% of the monomers containing hydrogen as $R^2$,
as a hair strengthening component in a water-based hair shampoo containing 1 to 30% by weight water-soluble foaming surfactants.

## Revendications

**1.** Shampooing fixateur sous forme de préparation aqueuse comprenant

| | |
|---|---|
| 1-30 % | en poids d'un tensioactif moussant, hydrosoluble, et |
| 0,1 - 10 % | en poids d'un polymère filmogène dispersé dans celui-ci, qui représente un homo- ou copolymère formé d'unités monomères de formule (I), |

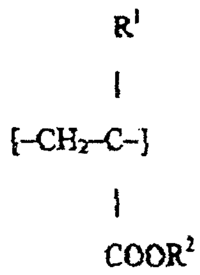

(I)

dans laquelle $R^1$ représente un atome d'hydrogène ou un groupe méthyle et $R^2$ représente un atome d'hydrogène ou un groupe alkyle comprenant de 1 à 8 atomes de C,
**caractérisé en ce que** pas plus de 60 % en moles des monomères contiennent de l'hydrogène en tant que $R^2$.

**2.** Shampooing fixateur selon la revendication 1, **caractérisé en ce que**, comme polymère filmogène, il contient un copolymère formé d'unités monomères de formule I, dans laquelle $R^1$ représente un atome d'hydrogène ou un groupe méthyle et $R^2$ représente un groupe alkyle ou éthyle et que la masse molaire moyenne est supérieure à 700 000.

**3.** Shampooing fixateur selon la revendication 1, **caractérisé en ce que**, comme polymère filmogène, il contient un copolymère formé d'unités monomères de formule I, dans laquelle $R^1$ représente un atome d'hydrogène ou un groupe méthyle et $R^2$ représente un atome d'hydrogène ou un groupe méthyle ou éthyle, **caractérisé en ce que** 40-60 % en moles des monomères contiennent de l'hydrogène en tant que $R^2$ et que la masse molaire moyenne est supérieure à 100 000.

**4.** Shampooing fixateur selon l'une quelconque des revendications 1-3, **caractérisé en ce qu'**il contient un solvant organique dans une proportion inférieure à 2,5 % en poids par rapport au poids total du shampooing.

**5.** Shampooing fixateur selon l'une quelconque des revendications 1-4, **caractérisé en ce que**, comme tensioactif moussant, hydrosoluble, il contient un tensioactif anionique, un tensioactif de type alkyl(oligo)glucoside ou un mélange de tensioactifs de ce type.

**6.** Shampooing fixateur selon la revendication 5, **caractérisé en ce que**, comme tensioactif anionique, il contient un tensioactif de type alkylsulfate ou alkylpolyglycoléthersulfate dont le groupe alkyle comprend 10-16 atomes de C, et jusqu'à 10 groupes glycoléther sous forme de sel de métal alcalin, de sel de magnésium, de sel d'ammonium ou de sel d'alcanolammonium.

**7.** Utilisation d'un polymère filmogène dispersé, qui représente un homo-ou copolymère formé d'unités monomères de formule (I),

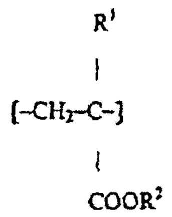

(I)

dans laquelle $R^1$ représente un atome d'hydrogène ou un groupe méthyle et $R^2$ représente un atome d'hydrogène ou un groupe alkyle comprenant de 1 à 8 atomes de C, pas plus de 60 % en moles des monomères contenant de l'hydrogène en tant que $R^2$,
en tant que composant fixateur capillaire dans un shampooing aqueux ayant une teneur de 1-30 % en poids de tensioactif moussant, hydrosoluble.